(19) Europäisches Patentamt
European Patent Office
Office européen des brevets

(11) EP 3 970 699 A1

(12) **EUROPEAN PATENT APPLICATION**
published in accordance with Art. 153(4) EPC

(43) Date of publication:
**23.03.2022 Bulletin 2022/12**

(21) Application number: **20815596.0**

(22) Date of filing: **29.05.2020**

(51) International Patent Classification (IPC):
***A61K 9/16*** *(2006.01)* ***A61K 9/00*** *(2006.01)*
***A61K 31/4725*** *(2006.01)* ***A61P 19/02*** *(2006.01)*
***A61P 29/00*** *(2006.01)*

(52) Cooperative Patent Classification (CPC):
**A61K 9/00; A61K 9/16; A61K 31/4725; A61P 19/02; A61P 19/08; A61P 29/00**

(86) International application number:
**PCT/KR2020/006954**

(87) International publication number:
**WO 2020/242235 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2019 KR 20190064301**

(71) Applicant: **LG Chem, Ltd.**
**Seoul 07336 (KR)**

(72) Inventors:
- **KIM, Sung Won**
  **Seoul 07796 (KR)**
- **KIM, Bok Tae**
  **Seoul 07796 (KR)**
- **CHOI, Sei Hyun**
  **Seoul 07796 (KR)**
- **BAEK, Jae Uk**
  **Seoul 07796 (KR)**

(74) Representative: **Hoffmann Eitle**
**Patent- und Rechtsanwälte PartmbB**
**Arabellastraße 30**
**81925 München (DE)**

# (54) COMPOSITION FOR CASPASE INHIBITOR PRODRUG INJECTION

(57) The present invention relates to a pharmaceutical composition for a caspase inhibitor prodrug injection and, more particularly, to a pharmaceutical composition for injection, the composition comprising a caspase inhibitor prodrug or a pharmaceutically acceptable salt or isomer thereof as an active ingredient, and a biocompatible polymer.

[Fig. 1]

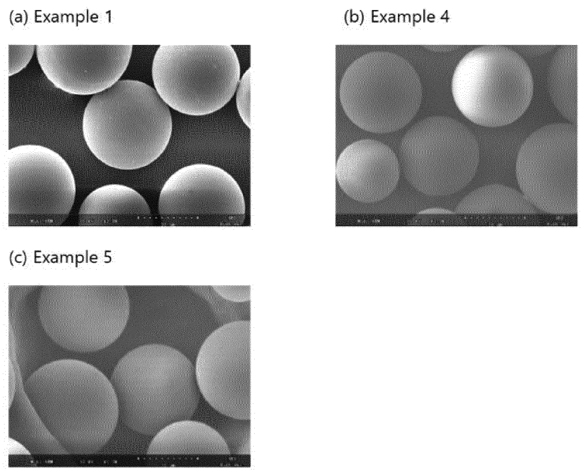


EP 3 970 699 A1

## Description

### TECHNICAL FIELD

The present invention relates to a pharmaceutical composition for injection of a caspase inhibitor prodrug. More specifically, the present invention relates to a pharmaceutical composition for injection comprising a prodrug of a caspase inhibitor, or a pharmaceutically acceptable salt or isomer thereof as an active ingredient, and a biocompatible polymer.

### BACKGROUND ART

Caspases are a type of enzymes and are cysteine proteases that exist as an $\alpha 2\beta 2$ tetramer. Caspase inhibitors interfere with the activity of these caspases, thereby regulating inflammation or apoptosis caused by the action of caspases. Diseases in which symptoms can be eliminated or alleviated by administration of these compounds include osteoarthritis, rheumatoid arthritis, degenerative arthritis, destructive bone disorder, hepatic diseases caused by hepatitis virus, acute hepatitis, hepatocirrhosis, brain damage caused by hepatitis virus, human fulminant liver failure, sepsis, organ transplantation rejection, ischemic cardiac disease, dementia, stroke, brain impairment due to AIDS, diabetes, gastric ulcer, etc.

Among compounds having various structures known as caspase inhibitors, isoxazoline derivatives were filed as Korean Patent Application Nos. 10-2004-0066726, 10-2006-0013107 and 10-2008-0025123. In addition, a prodrug of a caspase inhibitor based on an isoxazoline derivative was disclosed in International Publication No. WO 2007/015931 (Applicant: Vertex Pharmaceuticals Incorporated, USA).

Meanwhile, nivocasan ((R)-N-((2S,3 S)-2-(fluoromethyl)-2-hydroxy-5-oxotetrahydrofuran-3-yl]-5-isopropyl-3-(isoquinolin-1-yl)-4,5-dihydroisoxazole-5-carboxamide) of the following Formula 2 is attracting attention as an effective caspase inhibitor.

[Formula 2]

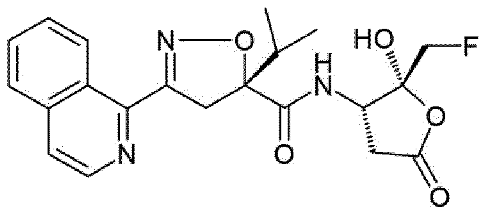

However, when nivocasan is prepared as a polymeric microsphere formulation in a sustained-release formulation, a large amount of drug is lost during the preparation process according to its physicochemical properties, resulting in low encapsulation efficiency, and there is a limit to *in vitro* drug release period.

### DISCLOSURE OF INVENTION

### TECHNICAL PROBLEM

Accordingly, the technical problem of the present invention is the provision of a composition for injection in which the encapsulation efficiency is greatly improved and the release period of the drug is greatly increased when a sustained-release formulation of a caspase inhibitor is prepared.

### SOLUTION TO PROBLEM

In order to solve the above technical problem, the present invention provides a pharmaceutical composition for injection comprising a compound of the following Formula 1, or a pharmaceutically acceptable salt or isomer thereof as an active ingredient; and a biocompatible polymer:

[Formula 1]

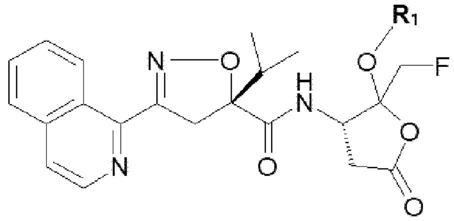

wherein

- $R_1$ represents alkyl, cycloalkyl, aryl or $-C(O)R_2$;
- $R_2$ represents alkyl, cycloalkyl, aryl, arylalkyl, or heteroaryl including one or more heteroatoms selected from N, O and S; and
- the alkyl, cycloalkyl, arylalkyl and heteroaryl are optionally substituted, and the substituent may be one or more selected from alkyl, cycloalkyl, hydroxy, halo, haloalkyl, acyl, amino, alkoxy, carboalkoxy, carboxy, carboxyamino, cyano, nitro, thiol, aryloxy, sulfoxy and guanido group.

In the present invention, the compound of Formula 1 may form a pharmaceutically acceptable salt. A pharmaceutically acceptable salt may include an acid-addition salt which is formed from an inorganic acid such as hydrochloric acid, sulfuric acid, nitric acid, phosphoric acid, hydrobromic acid and hydroiodic acid; an organic acid such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acid such as methanesulfonic acid, ethanesulfonic acid, benzenesulfonic acid and p-toluenesulfonic acid, which form non-toxic acid-addition salt including pharmaceutically acceptable anion. In addition, a pharmaceutically acceptable carboxylic acid salt includes the salt with alkali metal or alkali earth metal such as lithium, sodium, potassium, calcium and magnesium; salts with amino acid such as lysine, arginine and guanidine; an organic salt such as dicyclohexylamine, N-methyl-D-glucamine, tris(hydroxymethyl)methylamine, diethanolamine, choline and triethylamine. The compound of Formula 1 according to the present invention may be converted into their salts by conventional methods.

Meanwhile, since the compound of Formula 1 according to the present invention can have an asymmetric carbon center and asymmetric axis or plane, they can exist as E- or Z-isomer, R- or S-isomer, racemic mixtures or diastereoisomer mixtures and each diastereoisomer, all of which are within the scope of the present invention.

Herein, unless indicated otherwise, the term "the compound of Formula 1" is used to mean all the compounds of Formula 1, including the pharmaceutically acceptable salts and isomers thereof.

Herein, the following concepts defining the substituents are used to define the compound of Formula 1.

The term "halogen" or "halo" means fluoride (F), chlorine (Cl), bromine (Br) or iodine (I).

The term "alkyl" means straight or branched hydrocarbons, may include a single bond, a double bond or a triple bond, and is preferably $C_1$-$C_{10}$ alkyl. Examples of alkyl include, but are not limited to, methyl, ethyl, *n*-propyl, *i*-propyl, *n*-butyl, *i*-butyl, tert-butyl, pentyl, hexyl, heptyl, octyl, decyl, dodecyl, pentadecyl, octadecyl, acetylene, vinyl, trifluoromethyl and the like.

The term "cycloalkyl" means partially or fully saturated single or fused ring hydrocarbons, and is preferably $C_3$-$C_{10}$ cycloalkyl. Examples of cycloalkyl include, but are not limited to, cyclopropyl, cyclobutyl, cyclopentyl, cyclohexyl, cycloheptyl and the like.

Unless otherwise defined, the term "alkoxy" means alkyloxy having 1 to 10 carbon atoms.

The term "aryl" means aromatic hydrocarbons, preferably $C_5$-$C_{12}$ aryl, and more preferably $C_6$-$C_{10}$ aryl. Examples of aryl include, but are not limited to, phenyl, naphthyl and the like.

The term "heteroaryl" means 3- to 12-membered, more preferably 5- to 10-membered aromatic hydrocarbons which form a single or fused ring-which may be fused with benzo or $C_3$-$C_8$ cycloalkyl-including one or more heteroatoms selected from N, O and S as a ring member. Examples of heteroaryl include, but are not limited to, pyridinyl, pyrimidinyl, pyridazinyl, pyrazinyl, oxadiazolyl, isoxadiazolyl, tetrazolyl, triazolyl, indolyl, indazolyl, isoxazolyl, oxazolyl, thiazolyl, isothiazolyl, furanyl, benzofuranyl, imidazolyl, thiophenyl, benzthiazole, benzimidazole, quinolinyl, indolinyl, 1,2,3,4-tetrahydroisoquinolyl, 3,4-dihydroisoquinolinyl, thiazolopyridyl, 2,3-dihydrobenzofuran, 2,3-dihydrothiophene, 2,3-dihydroindole, benzo[1,3]dioxin, chroman, thiochroman, 1,2,3,4-tetrahydroquinoline, 4H-benzo[1,3]dioxin, 2,3-dihydrobenzo[1,4]-dioxin, 6,7-dihydro-5H-cyclopenta[d]pyrimidine and the like.

Aryl-alkyl, alkyl-aryl and heteroaryl-alkyl mean groups which are formed by the combination of the above-mentioned aryl and alkyl, or heteroaryl and alkyl. Examples include, but are not limited to, benzyl, thiophenemethyl,

pyrimidinemethyl and the like.

According to one embodiment of the present invention, in Formula 1 $R_1$ represents $C_1$-$C_8$ alkyl or -C(O)$R_2$; and $R_2$ represents $C_1$-$C_{20}$ alkyl, $C_6$-$C_{10}$ aryl or $C_6$-$C_{10}$ aryl-$C_1$-$C_7$ alkyl.

According to another embodiment of the present invention, in Formula 1 $R_1$ represents $C_1$-$C_5$ alkyl or -C(O)$R_2$; $R_2$ represents $C_1$-$C_{15}$ alkyl, $C_6$-$C_{10}$ aryl or $C_6$-$C_{10}$ aryl-$C_1$-$C_5$ alkyl; and the substituent is alkyl or haloalkyl.

Representative compounds of Formula 1 according to the present invention include, but are not limited to, the following compounds:

(2S,3S)-2-(fluoromethyl)-3 -((R)-5 -isopropyl-3 -(isoquinolin-1 -yl)-4,5 - dihydroisoxazole-5-carboxamido)-5-oxotetrahydrofuran-2-yl acetate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl propionate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl isobutyrate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl pivalate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 3-methylbutanoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 3,3-dimethylbutanoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl palmitate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl benzoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 4-(trifluoromethyl)benzoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 2-phenylacetate;
(5R)-N-((3S)-2-ethoxy-2-(fluoromethyl)-5-oxotetrahydrofuran-3-yl)-5-isopropyl-3-(isoquinolin-1-yl)-4,5-dihydroisoxazole-5-carboxamide; and
(5R)-N-((3S)-2-(fluoromethyl)-2-methoxy-5-oxotetrahydrofuran-3-yl)-5-isopropyl-3-(isoquinolin-1-yl)-4,5-dihydroisoxazole-5-carboxamide.

The terms and abbreviations used herein retain their original meanings unless indicated otherwise.

In one embodiment of the present invention, the biocompatible polymer may be one or more selected from the group consisting of polylactide (PLA), polyglycolide (PGA), poly(lactide-co-glycolide)(PLGA), polycaprolactone, polyorthoester and polyphosphazine, but is not limited thereto.

In one embodiment of the present invention, the biocompatible polymer is poly(lactide-co-glycolide). Poly(lactide-co-glycolide) may be polymerized from lactide and glycolide by ring-opening polymerization in the presence of a catalyst.

In one embodiment of the present invention, a molar ratio of lactide to glycolide of the poly(lactide-co-glycolide) is preferably 90:10 to 10:90, more preferably 90:10 to 40:60, and more preferably 85:15 to 50:50.

Poly(lactide-co-glycolide)-that is a polymer obtained by polymerizing lactic acid and glycolic acid, which are materials in the body-has biocompatibility and biodegradability, so it is widely used in medical and pharmaceutical fields through controlled-release of drugs. The higher the ratio of glycolide in poly(lactide-co-glycolide), the greater the hydrophilicity, so that the decomposition rate in the body is faster since moisture is absorbed well, and hydrolysis is accelerated. Conversely, as the ratio of lactide increases, hydrophobicity increases and moisture is not absorbed well, so that the resistance to hydrolysis increases and the decomposition rate in the body is delayed. In addition, the larger the average molecular weight of poly(lactide-co-glycolide), the longer the chain length of the polymer and the longer the decomposition time. Furthermore, the decomposition rate of poly(lactide-co-glycolide) may be affected by the type of end group, molecular structure, crystallinity and the like. In one embodiment of the present invention, the pharmaceutical composition for injection may further comprise a solvent. Examples of the solvent include, but are not limited to, water, saline or phosphate-buffered saline.

The injectable pharmaceutical composition of the present invention may further comprise other ingredients such as a dispersing agent, a wetting agent or a suspending agent, if necessary.

Exemplary diseases that can be prevented or treated by the pharmaceutical composition for injection according to the present invention include, but are not limited to, those selected from apoptosis-associated diseases, inflammatory diseases, osteoarthritis, rheumatoid arthritis, degenerative arthritis and destructive bone disorders.

In one embodiment of the present invention, the pharmaceutical composition for injection according to the present invention may be used for the prevention, treatment or pain relief of osteoarthritis.

## ADVANTAGEOUS EFFECTS OF INVENTION

In the present invention, by providing a pharmaceutical composition for injection in which the compound of Formula 1-which is a prodrug of a caspase inhibitor-is used, the encapsulation efficiency can be greatly improved when preparing polymeric microspheres, and the release period of the drug can be remarkably increased.

## BRIEF DESCRIPTION OF DRAWINGS

Figure 1 is photographs taken with a scanning electron microscope (SEM) of the microspheres prepared in Examples 1, 4 and 5.
Figure 2 is a photograph taken with a scanning electron microscope (SEM) of the microspheres prepared in the Comparative Example.
Figure 3 is a graph showing the results of the *in vitro* dissolution test in Experimental Example 2.

## MODE FOR THE INVENTION

Hereinafter, the present invention will be described in more detail through preparation examples and examples. However, these examples are only illustrative, and the scope of the present invention is not limited thereto.

**Preparation Example** 1: **(2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl acetate**

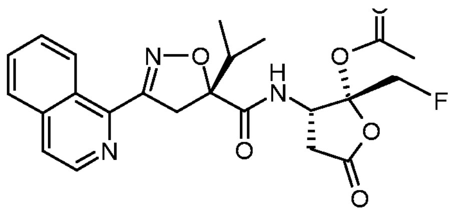

Nivocasan ((R)-N-((2S,3S)-2-(fluoromethyl)-2-hydroxy-5-oxotetrahydrofuran-3-yl]-5-isopropyl-3-(isoquinolin-1-yl)-4,5-dihydroisoxazole-5-carboxamide; 5.0 g, 12.0 mmol) was dissolved in dichloromethane (50 mL), and then acetyl chloride (0.94 mL, 13.2 mmol, 1.1 equiv), triethylamine (2.52 mL, 18.0 mmol, 1.5 equiv) and 4-dimethylaminopyridine (0.15 g, 1.2 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (25 mL). After adding water (25 mL) and stirring, the organic layer was separated and distilled under reduced pressure. The obtained mixture was recrystallized in a 1:5 mixture of ethyl acetate and hexane (EtOAc:hexane=1:5) to obtain 3.0 g (yield: 54%) of the title compound.

$^1H$ NMR (400 MHz, $CDCl_3$) $\delta$ 9.12 (d, 1H), 8.55 (d, 1H), 7.87 (d, 1H), 7.74-7.69 (m, 3H), 7.08 (d, 1H), 5.22 (m, 1H), 4.69 (d, 2H), 4.03 (d, 1H), 3.83 (d, 1H), 2.97 (m, 2H), 2.38 (m, 1H), 2.18 (s, 3H), 1.05 (dd, 6H)

**Preparation Example 2: (2S,3S)-2-(fluoromethyl)-3-((R)-5-isopropyl-3-(isoquinolin-1-yl)-4,5-dihydroisoxazote-5-carboxamido)-5-oxotetrahydrofuran-2-yl propionate**

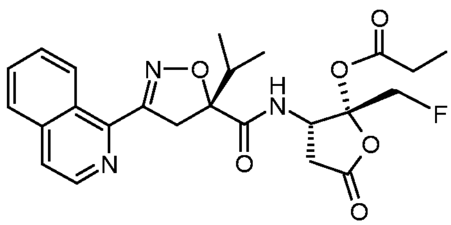

Nivocasan (1.0 g, 2.4 mmol) was dissolved in dichloromethane (20 mL), and then propionyl chloride (0.23 mL, 2.65 mmol, 1.1 equiv), triethylamine (0.5 mL, 3.61 mmol, 1.5 equiv) and 4-dimethylaminopyridine (0.03 g, 0.24 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was column separated by the use of a 1:2 mixture of ethyl acetate and hexane (EtOAc:hexane=1:2) to obtain 0.25 g (yield: 23%) of the title compound.

$^1$H NMR (400 MHz, $CDCl_3$) δ 9.12 (d, 1H), 8.55 (d, 1H), 7.87 (d, 1H), 7.74-7.69 (m, 3H), 7.08 (d, 1H), 5.22 (m, 1H), 4.69 (d, 2H), 4.03 (d, 1H), 3.83 (d, 1H), 2.98 (m, 2H), 2.45 (m, 2H), 2.37 (m, 1H), 1.20 (t, 3H), 1.05 (dd, 6H)

**Preparation Example 3: (2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl isobutyrate**

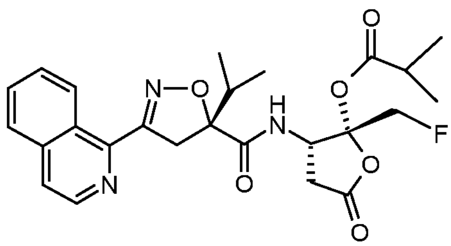

Nivocasan (1.0 g, 2.4 mmol) was dissolved in dichloromethane (20 mL), and then isobutyryl chloride (0.73 g, 2.65 mmol, 1.1 equiv), triethylamine (0.5 mL, 3.61 mmol, 1.5 equiv) and 4-dimethylaminopyridine (0.03 g, 0.24 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was column separated by the use of a 1:2 mixture of ethyl acetate and hexane (EtOAc:hexane=1:2) to obtain 0.06 g (yield: 5%) of the title compound.

$^1$H NMR (400 MHz, $CDCl_3$) δ 9.12 (d, 1H), 8.55 (d, 1H), 7.88 (d, 1H), 7.74-7.69 (m, 3H), 7.09 (d, 1H), 5.25 (m, 1H), 4.69 (d, 2H), 4.03 (d, 1H), 3.83 (d, 1H), 2.95 (m, 2H), 2.63 (m, 1H), 2.38 (m, 1H), 1.26 (dd, 6H), 1.05 (dd, 6H)

**Preparation Example 4: (2R,3S)-2-(fluoromethyl)-3-((R)-5-isopropyl-3-(isoquinolin-1-yl)-4,5-dihydroisoxazote-5-carboxamido)-5-oxotetrahydrofuran-2-yl pivalate**

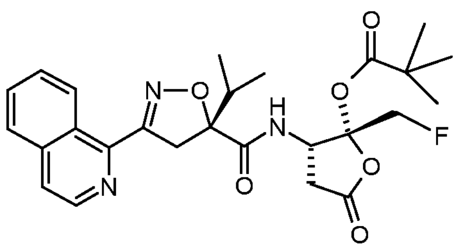

Nivocasan (0.5 g, 1.2 mmol) was dissolved in dichloromethane (20 mL), and then pivaloyl chloride (0.17 g, 1.4

mmol, 1.1 equiv) and 4-dimethylaminopyridine (0.29 g, 2.4 mmol, 2.0 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.1 g (yield: 17%) of the title compound.

$^1H$ NMR (400 MHz, $CDCl_3$) δ 9.11 (d, 1H), 8.55 (d, 1H), 7.88 (d, 1H), 7.71 (m, 3H), 7.11 (d, 1H), 5.26 (m, 1H), 4.68 (d, 2H), 4.02 (d, 1H), 3.83 (d, 1H), 2.95 (m, 2H), 2.39 (m, 1H), 2.27 (s, 2H), 1.28 (s, 9H), 1.05 (dd, 6H)

**Preparation Example 5: (2S,3S)-2-(fluoromethyl)-3-((R)-5-isopropyl-3-(isoquinolin-1-yl)-4,5-dihydroisoxazote-5-carboxamido)-5-oxotetrahydrofuran-2-yl 3-methylbutanoate**

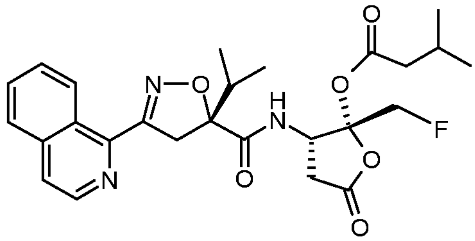

Nivocasan (0.5 g, 1.2 mmol) was dissolved in dichloromethane (20 mL), and then isovaleryl chloride (0.17 g, 1.4 mmol, 1.1 equiv), triethylamine (0.18 g, 1.8 mmol, 1.5 equiv) and 4-dimethylaminopyridine (0.015 g, 0.12 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.13 g (yield: 17%) of the title compound.

$^1H$ NMR (400 MHz, $CDCl_3$) δ 9.11 (d, 1H), 8.52 (d, 1H), 7.86 (d, 1H), 7.71 (m, 3H), 7.11 (d, 1H), 5.23 (m, 1H), 4.71 (d, 2H), 4.04 (d, 1H), 3.83 (d, 1H), 2.95 (m, 2H), 2.35 (m, 1H), 2.28 (d, 2H), 2.15 (m, 1H), 1.05 (dd, 12H)

**Preparation Example 6: (2S,3S)-2-(fluoromethyl)-3-((R)-5-isopropyl-3-(isoquinolin-1-yl)-4,5-dihydroisoxazote-5-carboxamido)-5-oxotetrahydrofuran-2-yl 3,3-dimethylbutanoate**

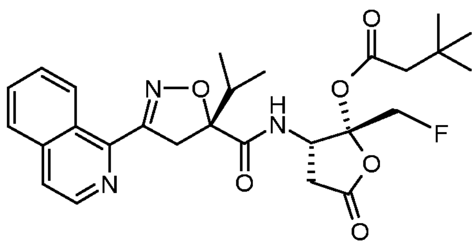

Nivocasan (0.5 g, 1.2 mmol) was dissolved in dichloromethane (20 mL), and then t-butyl acetyl chloride (0.19 g, 1.4 mmol, 1.1 equiv), triethylamine (0.18 g, 1.8 mmol, 1.5 equiv) and 4-dimethylaminopyridine (0.015 g, 0.12 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.14 g (yield: 23%) of the title compound.

$^1H$ NMR (400 MHz, $CDCl_3$) δ 9.12 (d, 1H), 8.55 (d, 1H), 7.88 (d, 1H), 7.71 (m, 3H), 7.13 (d, 1H), 5.23 (m, 1H), 4.71 (d, 2H), 4.04 (d, 1H), 3.83 (d, 1H), 2.95 (m, 2H), 2.35 (m, 1H), 2.27 (s, 2H), 1.05 (dd, 15H)

**Preparation Example 7: (2S, 3S)-2-(fluoromethyl)-3-((R)-5-isopropyl-3-(isoquinolin-1-yl)-4,5-dihydroisoxazote-5-carboxamido)-5-oxotetrahydrofuran-2-yl palmitate**

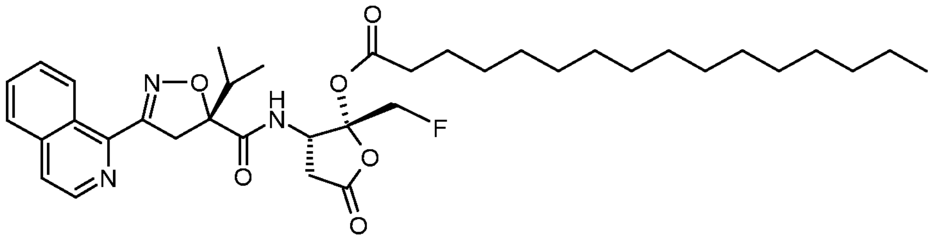

Nivocasan (1.0 g, 2.4 mmol) was dissolved in dichloromethane (20 mL), and then palmitoyl chloride (0.73 g, 2.65 mmol, 1.1 equiv), triethylamine (0.5 mL, 3.61 mmol, 1.5 equiv) and 4-dimethylaminopyridine (0.03 g, 0.24 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was column separated by the use of a 1:2 mixture of ethyl acetate and hexane (EtOAc:hexane=1:2) to obtain 0.11 g (yield: 7%) of the title compound.

$^1H$ NMR (400 MHz, $CDCl_3$) $\delta$ 9.12 (d, 1H), 8.55 (d, 1H), 7.88 (d, 1H), 7.74-7.69 (m, 3H), 7.09 (d, 1H), 5.23 (m, 1H), 4.69 (d, 2H), 4.03 (d, 1H), 3.83 (d, 1H), 2.95 (m, 2H), 2.41 (m, 2H), 2.38 (m, 1H), 1.68 (m, 2H), 1.35-1.24 (m, 24H), 1.05 (dd, 6H), 0.88 (t, 3H)

**Preparation Example 8: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl benzoate**

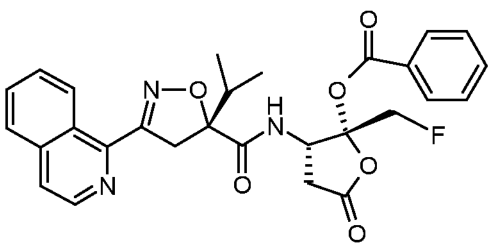

Nivocasan (0.5 g, 1.2 mmol) was dissolved in dichloromethane (20 mL), and then benzoyl chloride (0.17 g, 1.4 mmol, 1.1 equiv), triethylamine (0.18 g, 1.8 mmol, 1.5 equiv) and 4-dimethylaminopyridine (0.15 g, 1.2 mmol, 1.0 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.14 g (yield: 22%) of the title compound.

$^1H$ NMR (400 MHz, $CDCl_3$) $\delta$ 9.13 (d, 1H), 8.55 (d, 1H), 8.03 (d, 2H), 7.85 (d, 1H), 7.70 (m, 3H), 7.63 (t, 1H), 7.48 (m, 2H), 7.09 (d, 1H), 5.23 (m, 1H), 4.81 (d, 2H), 4.03 (d, 1H), 3.74 (d, 1H), 3.08 (m, 2H), 2.20 (m, 1H), 0.97 (dd, 6H)

**Preparation Example 9: (2S,3S)-2-(fluoromethyl)-3-((R)-5-isopropyl-3-(isoquinolin-1-yl)-4,5-dihydroisoxazole-5-carboxamido)-5-oxotetrahydrofuran-2-yl 4-(trifluoromethyl)benzoate**

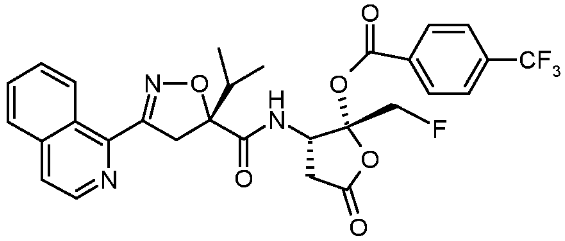

Nivocasan (0.5 g, 1.2 mmol) was dissolved in dichloromethane (20 mL), and then 4-trifluoromethyl benzoyl chloride (0.30 g, 1.4 mmol, 1.1 equiv) and 4-dimethylaminopyridine (0.29 g, 2.4 mmol, 2.0 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1 :5) to obtain 0.1 g (yield: 13%) of the title compound.

$^{1}H$ NMR (400 MHz, $CDCl_3$) δ 9.12 (d, 1H), 8.55 (d, 1H), 8.14 (d, 2H), 7.88 (d, 1H), 7.74 (m, 3H), 7.85 (m, 2H), 7.09 (d, 1H), 5.26 (m, 1H), 4.82 (d, 2H), 4.03 (d, 1H), 3.83 (d, 1H), 3.08 (m, 2H), 2.19 (m, 1H), 0.82 (dd, 6H)

**Preparation Example 10: (2S,3S)-2-(fluoromethyl)-3-((R)-5-isopropyl-3-(isoquinolin-1-yl)-4,5-dihydroisoxazote-5-carboxamido)-5-oxotetrahydrofuran-2-yl 2-phenylacetate**

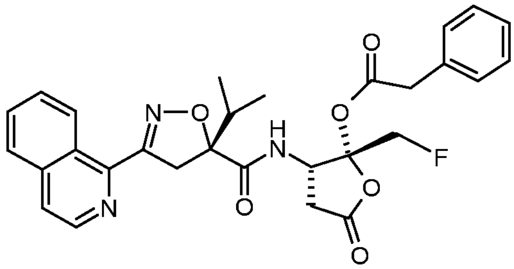

Nivocasan (0.5 g, 1.2 mmol) was dissolved in dichloromethane (20 mL), and then phenylacetyl chloride (0.22 g, 1.4 mmol, 1.1 equiv), triethylamine (0.18 g, 1.8 mmol, 1.5 equiv) and 4-dimethylaminopyridine (0.015 g, 0.12 mmol, 0.1 equiv) were added thereto while keeping the temperature of 5°C or lower. The reaction mixture was stirred at 25°C for about 2 hours, and the reaction was terminated by adding 10% aqueous sodium hydrogen carbonate solution (10 mL). After adding water (10 mL) and stirring, the organic layer was separated and distilled under reduced pressure. The obtained mixture was recrystallized in a 1:5 mixture of ethyl acetate and hexane (EtOAc:hexane=1:5) to obtain 0.3 g (yield: 51%) of the title compound.

$^{1}H$ NMR (400 MHz, $CDCl_3$) δ 9.12 (d, 1H), 8.55 (d, 1H), 7.88 (d, 1H), 7.74-7.69 (m, 3H), 7.37 (m, 2H), 7.30(m, 3H), 7.09 (d, 1H), 5.23 (m, 1H), 4.69 (d, 2H), 4.03 (d, 1H), 3.83 (d, 1H), 3.74 (s, 2H), 2.95 (m, 2H), 2.38 (m, 1H), 1.05 (dd, 6H)

**Preparation Example 11-1: (S)-4,4-diethoxy-5-fluoro-3-((R)-5-isopropyl-3-(isoquinolin-1-yl)-4,5-dihydroisoxazole-5-carboxamido)pentanoic acid**

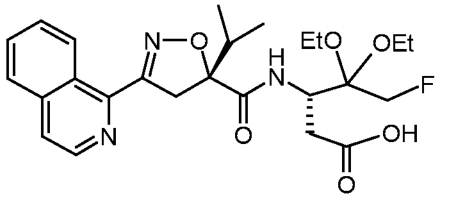

(Step A) Ethyl (S)-5-fluoro-3-((R)-5-isopropyl-3-(isoquinolin-1-yl)-4,5-dihydroisoxazole-5-carboxamido)-4-oxopentanoate

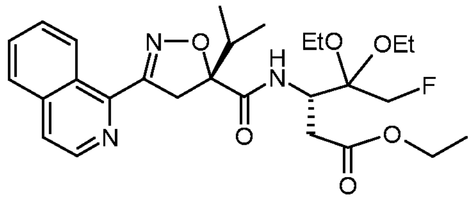

Nivocasan (500 mg, 1.2 mmol) was reacted with p-tosylic acid (114 mg, 0.6 mmol), triethoxymethane (20 ml, 120 mmol) and ethanol (20 ml) under reflux for 6 days. The reaction mixture was cooled to room temperature, saturated ammonium chloride solution was added thereto, and extracted with ethyl acetate. The organic layer was dried over sodium sulfate, concentrated and purified by the use of MPLC to obtain the title compound (230 mg, 37%).

$^1$H-NMR ($CDCl_3$) $\delta$ 9.15 (d, 1H), 8.54 (d, 1H), 7.84 (d, 1H), 7.75 ~ 7.64 (m, 3H), 4.75 ~ 4.86 (m, 1H) 4.53 (dd, 1H), 4.42 (dd, 1H), 4.02 (d, 1H) 3.97 ~ 3.67 (m, 5H), 3.57 ~ 3.50 (m, 3H), 2.71 (dd, 1H), 2.52 ~ 2.36 (m, 2H), 1.18 ~ 0.97 (m, 15H)

(Step B) (S)-4,4-diethoxy-5-fluoro-3-((R)-5-isopropyl-3-(isoquinolin-1-yl)-4,5-dihydroisoxazole-5-carboxamido)pentanoic acid

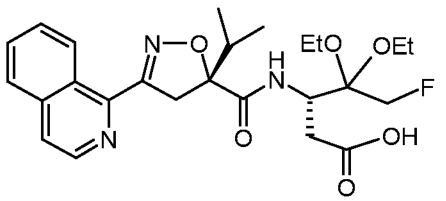

To ethyl (S)-5-fluoro-3-((R)-5-isopropyl-3-(isoquinolin-1-yl)-4,5-dihydroisooxazole-5-carboxamido)-4-oxopentanoate (230 mg, 0.44 mmol) and lithium hydroxide (31.9 mg, 1.33 mmol), water (1.12 ml) and tetrahydrofuran (THF, 0.28 ml) were added, and the reaction was carried out at 40°C for 3 hours. The reactant was cooled to room temperature, concentrated under reduced pressure, 1 N sodium hydroxide was added, and the water layer was washed with toluene. Then, 6N hydrochloric acid was added to adjust the pH to 3, followed by extraction using dichloromethane. The extracted organic layer was dried over sodium sulfate and filtered under reduced pressure to obtain the title compound. The obtained title compound was used in the next reaction without further purification.

**Preparation Example 11-2: (5R)-N-((3S)-2-ethoxy-2-(fluoromethyl)-5-oxotetrahydrofuran-3-yl)-5-isopropyl-3-(isoquinolin-1-yl)-4,5-dihydroisoxazole-5-carboxamide**

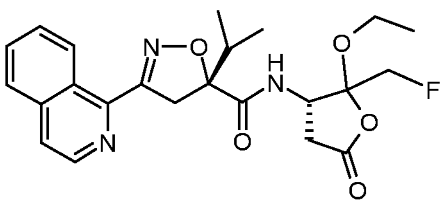

To (S)-4,4-diethoxy-5-fluoro-3-((R)-5-isopropyl-3-(isoquinolin-1-yl)-4,5-dihydroisooxazole-5-carboxamido)pentanoic acid (200 mg, 0.38 mmol), trifluoroacetic acid (0.5 ml) and dichloromethane (5 ml) were added at 0°C and stirred at room temperature for 2 hours. After stirring for 2 hours, the reaction mixture was concentrated under reduced

pressure and the title compound (92 mg, 54%) was obtained through MPLC.

$^1$H-NMR ($CDCl_3$) $\delta$ 9.11 (d, 1H), 8.56 (d, 1H), 7.86 (d, 1H), 7.75 ~ 7.64 (m, 3H), 7.38 (d, 1H) 4.94 (q, 1H) 4.66 (s, 1H), 4.54 (s, 1H), 4.09 (d, 1H), 4.02 (d, 1H) 3.90 ~ 3.67 (m, 3H), 2.92 (dd, 1H), 2.59 (dd, 1H), 2.36 (p, 1H), 1.29 ~ 1.19 (m, 4H), 1.06 (t, 6H)

**Preparation Example 12-1: (S)-5-fluoro-3-((R)-5-isopropyl-3-(isoquinolin-1-yl)-4,5-dihydroisoxazole-5-carboxamido)-4,4-dimethoxypentanoic acid**

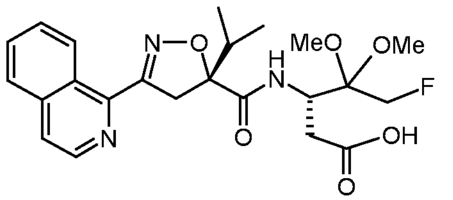

(Step A) Methyl (S)-5-fluoro-3-((R)-5-isopropyl-3-(isoquinolin-1-yl)-4,5-dihydroisoxazole-5-carboxamido)-4,4-dimethoxypentanoate

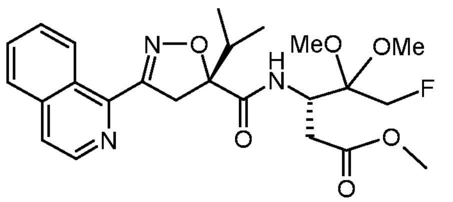

Nivocasan (1 g, 2.4 mmol) was reacted with p-tosylic acid (229 mg, 1.2 mmol), triethoxymethane (10 ml, 90 mmol) and methanol (20 ml) under reflux for 4 days. The reaction mixture was cooled to room temperature, saturated ammonium chloride solution was added thereto, and extracted with ethyl acetate. The organic layer was dried over sodium sulfate, concentrated and purified by the use of MPLC to obtain the title compound (561 mg, 49%).

(Step B) (S)-5-fluoro-3-((R)-5-isopropyl-3-(isoquinolin-1-yl)-4,5-dihydroisoxazole-5-carboxamido)-4,4-dimethoxypentanoic acid

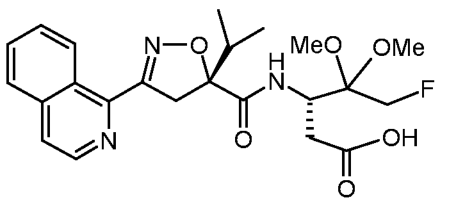

To methyl (S)-5-fluoro-3-((R)-5-isopropyl-3-(isoquinolin-1-yl)-4,5-dihydroisoxazole-5-carboxamido)-4,4-dimethoxypentanoate (562 mg, 1.18 mmol) and lithium hydroxide (134 mg, 5.59 mmol), water (3.42 ml) and THF (0.85 ml) were added, and the reaction was carried out at 40°C for 4 hours. The reactant was cooled to room temperature, concentrated under reduced pressure, 1 N sodium hydroxide was added, and the water layer was washed with toluene. Then, 6N hydrochloric acid was added to adjust the pH to 3, followed by extraction using dichloromethane. The extracted organic layer was dried over sodium sulfate and filtered under reduced pressure to obtain the title compound. The obtained title compound was used in the next reaction without further purification.

**Preparation Example** 12-2: **<u>(5R)-N-((3S)-2-(fluoromethyl)-2-methoxy-5-oxotetrahydrofuran-3-yl)-5-isopropyl-3-(isoquinolin-1-yl)-4,5-dihydroisoxazole-5-carboxamide</u>**

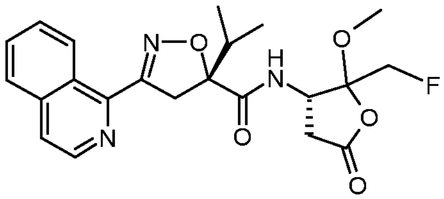

To (S)-5-fluoro-3-((R)-5-isopropyl-3-(isoquinolin-1-yl)-4,5-dihydroisoxazole-5-carboxamido)-4,4-dimethoxypentanoic acid (441 mg, 0.9 mmol), trifluoroacetic acid (0.5 ml) and dichloromethane (5 ml) were added at 0°C and and stirred at room temperature for 2 hours. After stirring for 2 hours, the mixture was concentrated under reduced pressure and the title compound (196 mg, 51%) was obtained through MPLC.

$^1$H-NMR ($CDCl_3$) δ 9.12 (d, 1H), 8.56 (d, 1H), 7.86 (d, 1H), 7.75 ~ 7.64 (m, 3H), 7.40 (d, 1H) 4.91 (q, 1H) 4.66 (dd, 1H), 4.54 (dd, 1H), 4.08 (d, 1H), 4.80 (d, 1H), 3.35 (s, 3H), 2.90 (dd, 1H), 2.56 (dd, 1H), 2.36 (p, 1H), 1.06 (t, 6H)

**<u>Examples 1 to 6: Preparation of microspheres encapsulating prodrugs</u>**

According to the compositions denoted in Table 1 below, microspheres encapsulated with caspase inhibitor prodrugs were prepared.

The caspase inhibitor prodrugs and PLGA (L/G ratio = 50:50, 75:25 or 85:15, M.W. 38,000 - 240,000) were weighed in a weight ratio of 1:5, an organic solvent dichloromethane was added in an amount of 10 times the weight of PLGA or the weight of the prodrug and PLGA, and stirred to prepare the disperse phase.

For the continuous phase, 150 mL or 4,800 mL of 1% or 2% polyvinyl alcohol (M.W. 31,000 - 50,000, degree of hydrolysis 87 - 89%) was used, and emulsions were prepared by membrane emulsification.

The prepared emulsions were stirred overnight at room temperature to remove solvent, washed with sterile purified water, and then lyophilized to obtain microspheres.

[Table 1]

| **Example No.** | **1** | **2** | **3** | **4** | **5** | **6** |
|---|---|---|---|---|---|---|
| **Prodrug** | Preparation Example 1 | Preparation Example 2 | Preparation Example 8 | Preparation Example 1 | Preparation Example 1 | Preparation Example 1 |
| **Prodrug amount (g)** | 0.4 | 0.4 | 0.4 | 3.4 | 3.4 | 2.5 |
| **PLGA amount (g)** | 2.0 | 2.0 | 2.0 | 17.0 | 17.0 | 12.5 |
| **PLGA L/G ratio** | 50:50 | 50:50 | 50:50 | 50:50 | 75:25 | 85:15 |
| **PLGA M.W. (kDa)** | 38-54 | 38-54 | 38-54 | 38-54 | 76-115 | 190-240 |
| **Organic solvent amount (g)** | 20.0 | 20.0 | 20.0 | 170.0 | 170.0 | 150.0 |
| **PVA concentration (%, w/v)** | 2 | 2 | 2 | 1 | 1 | 1 |
| **PVA solution amount (mL)** | 150 | 150 | 150 | 4,800 | 4,800 | 4,800 |

**Comparative Example: Preparation of microspheres encapsulating nivocasan**

According to the composition denoted in Table 2 below, microspheres encapsulated with nivocasan were prepared.
Nivocasan and PLGA (L/G ratio = 50:50, M.W. 38,000 - 54,000) were weighed in a weight ratio of 1:5, an organic solvent dichloromethane was added in an amount of 10 times the weight of PLGA, and stirred to prepare the disperse phase.
For the continuous phase, 150 mL of 2% polyvinyl alcohol (M.W. 31,000 - 50,000, degree of hydrolysis 87 - 89%) was used, and emulsions were prepared by membrane emulsification.
The prepared emulsions were stirred overnight at room temperature to remove solvent, washed with sterile purified water, and then lyophilized to obtain microspheres.

[Table 2]

| Compound | Nivocasan |
|---|---|
| Compound amount (g) | 0.4 |
| PLGA amount (g) | 2.0 |
| PLGA L/G ratio | 50:50 |
| PLGAM.W. (kDa) | 38-54 |
| Organic solvent amount (g) | 20.0 |
| PVA concentration (%, w/v) | 2 |
| PVA solution amount (mL) | 150 |

**Experimental Example 1: Analysis** of microsphere **properties and drug encapsulation rate**

The properties of the microspheres prepared in the Examples and Comparative Example were characterized by drug precipitation during manufacture, the morphology of lyophilized microspheres, and floating in the aqueous phase upon redispersion.
Whether or not precipitation of the drug was confirmed through an optical microscope during manufacture, and the morphology of the lyophilized microspheres were observed by scanning electron microscopy. Whether or not floating of the microspheres in the aqueous phase was confirmed by redispersing the lyophilized microspheres in water.
For the amount of drug encapsulated in the microspheres, 30 mg of microspheres were dissolved in 50 mL of acetonitrile, and the supernatant obtained by ultracentrifugation was analyzed by HPLC (high-performance liquid chromatography). The encapsulation efficiency was calculated by measuring the encapsulation rate.

- Encapsulation rate = (weight of measured drug)/(weight of measured microspheres (MS))*100 (%)

- Encapsulation efficiency = (weight of measured drug)/(weight of drug added initially) *100 (%)

The measured results are summarized and represented in Table 3.

[Table 3]

| | Example 1 | Example 4 | Exmaple 5 | Example 6 | Comp. Ex. |
|---|---|---|---|---|---|
| Drug precipitation | Almost none | Almost none | Almost none | Almost none | Large amount |
| Microsphere morphology | Good | Good | Good | Good | Good |
| Microsphere floating | None | None | None | None | None |

(continued)

| | Example 1 | Example 4 | Exmaple 5 | Example 6 | Comp. Ex. |
|---|---|---|---|---|---|
| Drug precipitation | Almost none | Almost none | Almost none | Almost none | Large amount |
| Drug encapsulation rate (%, w/w) | 15.6 | 14.4 | 13.9 | 14.5 | 8.2 |
| Drug encapsulation efficiency (%) | 93.4 | 86.4 | 83.4 | 87.0 | 49.1 |

As can be seen from Table 3, the microspheres of the Examples encapsulating the prodrugs showed little drug precipitation and excellent drug encapsulation efficiency, whereas in the microspheres of the Comparative Example encapsulating nivocasan, a large amount of the drug was precipitated during the preparation process, and the drug encapsulation efficiency was only about half in comparison with the Examples.

**Experimental Example 2: *In vitro* dissolution test of microspheres**

An *in vitro* dissolution test of the microspheres prepared in Examples 4 and 5 was carried out. The microspheres were shaken in phosphate-buffered saline (PBS, 37°C), the eluate was collected and filtered at specific times, and the amount of drug released was analyzed by HPLC. Because the prodrug is converted to the parent drug, caspase inhibitor, by hydrolysis in aqueous solution, the amount of the released drug was confirmed through the amount of caspase inhibitor measured by HPLC.

## Claims

1. A pharmaceutical composition for injection comprising a compound of the following Formula 1, or a pharmaceutically acceptable salt or isomer thereof as an active ingredient; and a biocompatible polymer:

[Formula 1]

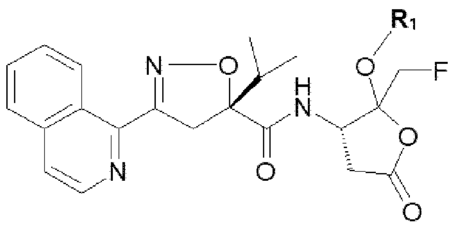

wherein

$R_1$ represents alkyl, cycloalkyl, aryl or -C(O)$R_2$;
$R_2$ represents alkyl, cycloalkyl, aryl, arylalkyl, or heteroaryl including one or more heteroatoms selected from N, O and S; and
the alkyl, cycloalkyl, arylalkyl and heteroaryl are optionally substituted, and the substituent may be one or more selected from alkyl, cycloalkyl, hydroxy, halo, haloalkyl, acyl, amino, alkoxy, carboalkoxy, carboxy, carboxyamino, cyano, nitro, thiol, aryloxy, sulfoxy and guanido group.

2. The pharmaceutical composition for injection according to Claim 1, wherein

$R_1$ represents $C_1$-$C_8$ alkyl or -C(O)$R_2$; and
$R_2$ represents $C_1$-$C_{20}$ alkyl, $C_6$-$C_{10}$ aryl or $C_6$-$C_{10}$ aryl-$C_1$-$C_7$ alkyl.

3. The pharmaceutical composition for injection according to Claim 1, wherein

$R_1$ represents $C_1$-$C_5$ alkyl or -C(O)$R_2$;
$R_2$ represents $C_1$-$C_{15}$ alkyl, $C_6$-$C_{10}$ aryl or $C_6$-$C_{10}$ aryl-$C_1$-$C_5$ alkyl; and

the substituent is alkyl or haloalkyl.

4. The pharmaceutical composition for injection according to Claim 1, wherein the compound of Formula 1 is selected from the following group:

(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl acetate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl propionate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl isobutyrate;
(2R,3S)-2-(fluoromethyl)-3-((R)-5-isopropyl-3-(isoquinolin-1-yl)-4,5-dihydroisoxazole-5-carboxamido)-5-oxotetrahydrofuran-2-yl pivalate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 3-methylbutanoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 3,3-dimethylbutanoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl palmitate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl benzoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 4-(trifluoromethyl)benzoate;
(2S,3S)-2-(fluoromethyl)-3 -((R)-5 -isopropyl-3 -(isoquinolin-1-yl)-4,5-dihydroisoxazole-5-carboxamido)-5-oxotetrahydrofuran-2-yl 2-phenylacetate;
(5R)-N-((3S)-2-ethoxy-2-(fluoromethyl)-5-oxotetrahydrofuran-3-yl)-5-isopropyl-3-(isoquinolin-1-yl)-4,5-dihydroisoxazole-5-carboxamide; and
(5R)-N-((3S)-2-(fluoromethyl)-2-methoxy-5-oxotetrahydrofuran-3-yl)-5-isopropyl-3-(isoquinolin-1-yl)-4,5-dihydroisoxazole-5-carboxamide.

5. The pharmaceutical composition for injection according to Claim 1, wherein the biocompatible polymer is one or more selected from the group consisting of polylactide, polyglycolide, poly(lactide-co-glycolide), polycaprolactone, polyorthoester and polyphosphazine.

6. The pharmaceutical composition for injection according to Claim 5, wherein the biocompatible polymer is poly(lactide-co-glycolide).

7. The pharmaceutical composition for injection according to Claim 6, wherein a molar ratio of lactide to glycolide of the poly(lactide-co-glycolide) is 90:10 to 10:90.

8. The pharmaceutical composition for injection according to Claim 7, wherein a molar ratio of lactide to glycolide of the poly(lactide-co-glycolide) is 90:10 to 40:60.

9. The pharmaceutical composition for injection according to Claim 8, wherein a molar ratio of lactide to glycolide of the poly(lactide-co-glycolide) is 85:15 to 50:50.

10. The pharmaceutical composition for injection according to Claim 1, which further comprises a solvent.

11. The pharmaceutical composition for injection according to Claim 10, wherein the solvent is water, saline or phosphate-buffered saline.

12. The pharmaceutical composition for injection according to Claim 1, which is for the prevention or treatment of a disease selected from apoptosis-associated diseases, inflammatory diseases, osteoarthritis, rheumatoid arthritis, degenerative arthritis and destructive bone disorders.

13. The pharmaceutical composition for injection according to Claim 12, which is for the prevention, treatment or pain relief of osteoarthritis.

[Fig. 1]

(a) Example 1

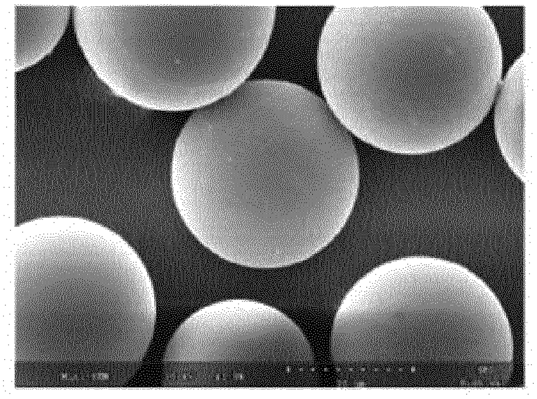

(b) Example 4

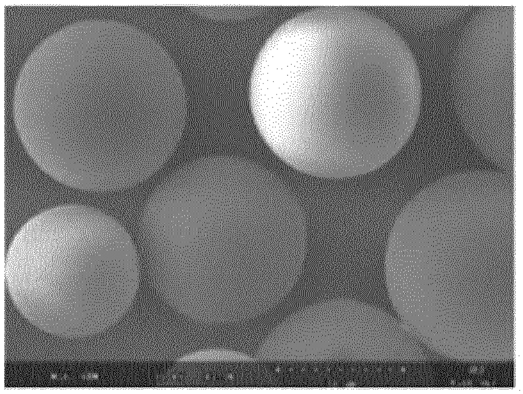

(c) Example 5

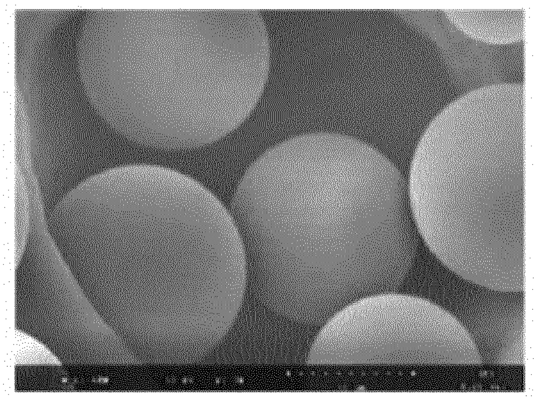

[Fig. 2]

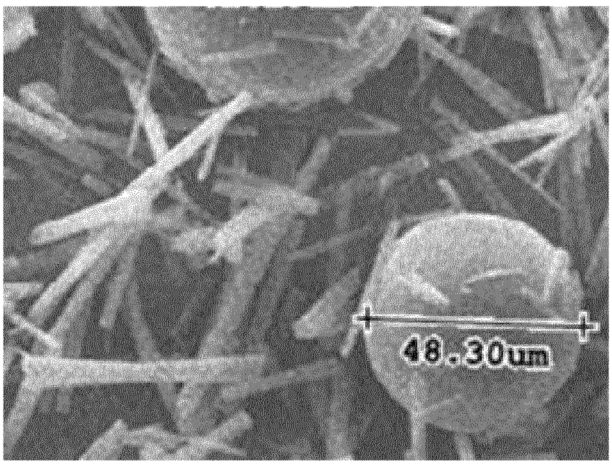

[Fig. 3]

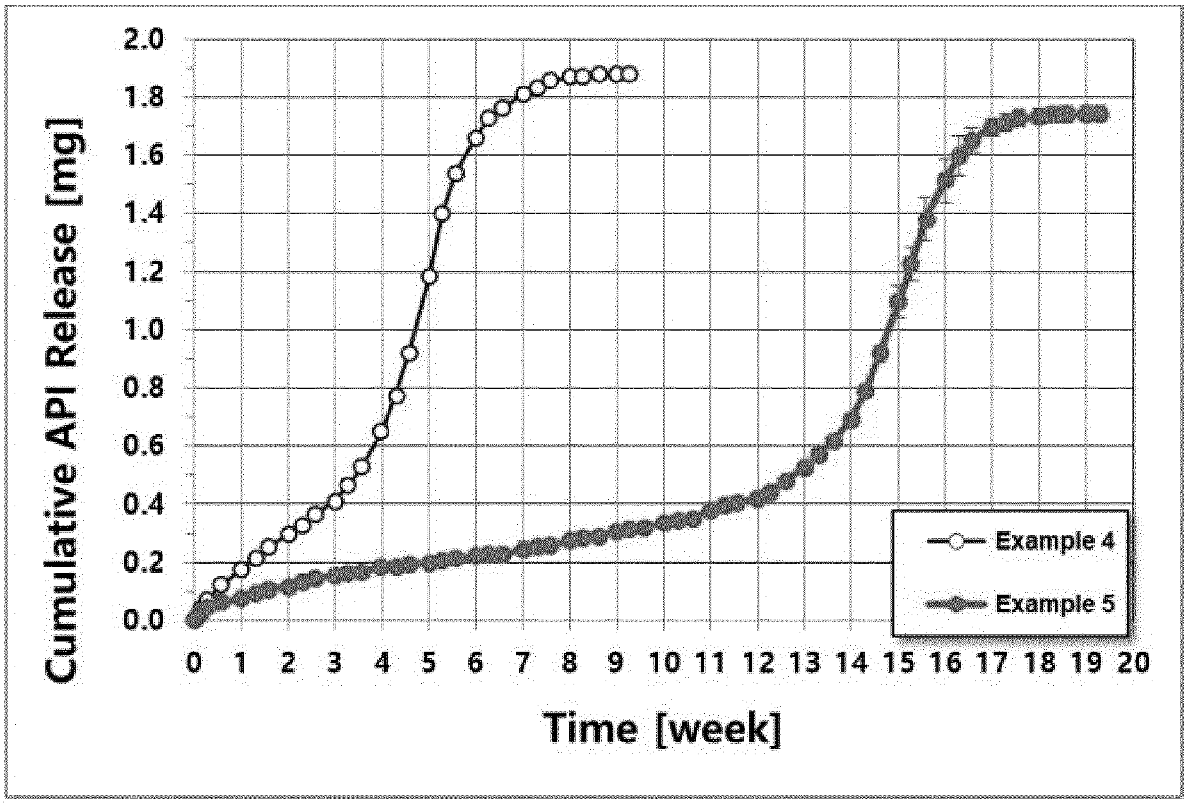
Cumulative API Release [mg]
2.0
1.8
1.6
1.4
1.2
1.0
0.8
0.6
0.4
0.2
0.0
0 1 2 3 4 5 6 7 8 9 10 11 12 13 14 15 16 17 18 19 20
Time [week]
Example 4
Example 5

**INTERNATIONAL SEARCH REPORT**

International application No.
PCT/KR2020/006954

A. CLASSIFICATION OF SUBJECT MATTER

*A61K 9/16(2006.01)i, A61K 9/00(2006.01)i, A61K 31/4725(2006.01)i, A61P 19/02(2006.01)i, A61P 29/00(2006.01)i*

According to International Patent Classification (IPC) or to both national classification and IPC

B. FIELDS SEARCHED

Minimum documentation searched (classification system followed by classification symbols)
A61K 9/16; A01N 43/80; A61K 31/138; A61K 31/325; A61K 31/4725; A61P 1/16; C07D 413/04; C07D 413/12; C07D 413/14; A61K 9/00; A61P 19/02; A61P 29/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), STN (Registry, Caplus), Google & Keywords: isoxazoline derivatives, isoquinolinyl group, caspase, lactone, injection

C. DOCUMENTS CONSIDERED TO BE RELEVANT

| Category* | Citation of document, with indication, where appropriate, of the relevant passages | Relevant to claim No. |
|---|---|---|
| DA | KR 10-2006-0094868 A (LG LIFE SCIENCES LTD.) 30 August 2006<br>See claims 1-3, 26. | 1-13 |
| A | WO 2010-005765 A1 (GILEAD SCIENCES, INC.) 14 January 2010<br>See the entire document. | 1-13 |
| A | US 2010-0120843 A1 (CHANG, H. K. et al.) 13 May 2010<br>See the entire document. | 1-13 |
| A | WO 2017-059427 A1 (CHILDREN'S NATIONAL MEDICAL CENTER) 06 April 2017<br>See the entire document. | 1-13 |
| A | WO 2014-048940 A2 (BAYER CROPSCIENCE AG.) 03 April 2014<br>See the entire document. | 1-13 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 SEPTEMBER 2020 (02.09.2020) | **03 SEPTEMBER 2020 (03.09.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.
PCT/KR2020/006954

| Patent document cited in search report | Publication date | Patent family member | Publication date |
|---|---|---|---|
| KR 10-2006-0094868 A | 30/08/2006 | AP 2374 A | 07/03/2012 |
| | | AR 055314 A1 | 15/08/2007 |
| | | AU 2006-217293 A1 | 31/08/2006 |
| | | AU 2006-217293 B2 | 02/02/2012 |
| | | BR PI0607330 A2 | 29/09/2009 |
| | | CA 2598347 A1 | 31/08/2006 |
| | | CA 2598347 C | 02/08/2011 |
| | | CN 101128459 A | 20/02/2008 |
| | | CN 101128459 B | 05/10/2011 |
| | | DK 1851214 T3 | 15/10/2012 |
| | | EA 013005 B1 | 26/02/2010 |
| | | EA 200701810 A1 | 28/02/2008 |
| | | EP 1851214 A1 | 07/11/2007 |
| | | EP 1851214 A4 | 28/10/2009 |
| | | EP 1851214 B1 | 05/09/2012 |
| | | HK 1111146 A1 | 14/12/2012 |
| | | JP 2008-531551 A | 14/08/2008 |
| | | JP 4961357 B2 | 27/06/2012 |
| | | KR 10-0774999 B1 | 09/11/2007 |
| | | MA 29311 B1 | 03/03/2008 |
| | | MX 2007010338 A | 11/10/2007 |
| | | MY 149181 A | 31/07/2013 |
| | | NO 20074895 A | 26/11/2007 |
| | | NO 341347 B1 | 16/10/2017 |
| | | NZ 560805 A | 24/12/2010 |
| | | PE 20061076 A1 | 27/12/2006 |
| | | PL 1851214 T3 | 29/03/2013 |
| | | PT 1851214 E | 18/10/2012 |
| | | SG 156689 A1 | 26/11/2009 |
| | | SI EP1851214 T1 | 30/11/2012 |
| | | TW 200640918 A | 01/12/2006 |
| | | TW I377205 B | 21/11/2012 |
| | | UA 94395 C2 | 10/05/2011 |
| | | US 2008-0262032 A1 | 23/10/2008 |
| | | US 8044080 B2 | 25/10/2011 |
| | | WO 2006-090997 A1 | 31/08/2006 |
| | | ZA 200707202 B | 29/10/2008 |
| WO 2010-005765 A1 | 14/01/2010 | TW 201012816 A | 01/04/2010 |
| | | US 2010-0008867 A1 | 14/01/2010 |
| US 2010-0120843 A1 | 13/05/2010 | None | |
| WO 2017-059427 A1 | 06/04/2017 | None | |
| WO 2014-048940 A2 | 03/04/2014 | AR 092727 A1 | 29/04/2015 |
| | | BR 15007159 A2 | 04/07/2017 |
| | | CN 104812751 A | 29/07/2015 |
| | | CN 104812751 B | 05/06/2018 |

Form PCT/ISA/210 (patent family annex) (January 2015)

INTERNATIONAL SEARCH REPORT
Information on patent family members

International application No.
PCT/KR2020/006954

| Patent document cited in search report | Publication date | Patent family member | Publication date |
|---|---|---|---|
| | | EP 2900661 A2 | 05/08/2015 |
| | | EP 2900661 B1 | 26/10/2016 |
| | | JP 2015-535834 A | 17/12/2015 |
| | | JP 6118907 B2 | 19/04/2017 |
| | | TW 201418249 A | 16/05/2014 |
| | | TW I579281 B | 21/04/2017 |
| | | US 2015-0216172 A1 | 06/08/2015 |
| | | US 2017-0057951 A1 | 02/03/2017 |
| | | US 9510598 B2 | 06/12/2016 |
| | | US 9776993 B2 | 03/10/2017 |
| | | WO 2014-048940 A3 | 22/05/2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020040066726
- KR 1020060013107
- KR 1020080025123
- WO 2007015931 A